(19) Europäisches Patentamt
European Patent Office
Office européen des brevets

(11) **EP 3 773 150 B1**

(12) **EUROPÄISCHE PATENTSCHRIFT**

(45) Veröffentlichungstag und Bekanntmachung des Hinweises auf die Patenterteilung:
**17.04.2024   Patentblatt 2024/16**

(21) Anmeldenummer: **18795923.4**

(22) Anmeldetag: **12.07.2018**

(51) Internationale Patentklassifikation (IPC):
**A61B 3/16** (2006.01)      **A61B 3/12** (2006.01)

(52) Gemeinsame Patentklassifikation (CPC):
**A61B 3/16; A61B 3/1241;** A61B 3/0008; A61B 3/12; A61B 3/1233; A61B 3/18

(86) Internationale Anmeldenummer:
**PCT/DE2018/100639**

(87) Internationale Veröffentlichungsnummer:
**WO 2019/185074 (03.10.2019 Gazette 2019/40)**

(54) **VORRICHTUNG ZUR UNTERSUCHUNG DER METABOLISCHEN AUTOREGULATION**

DEVICE FOR EXAMINING METABOLIC AUTOREGULATION

DISPOSITIF POUR ANALYSER L'AUTORÉGULATION MÉTABOLIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.03.2018   DE 102018107621**

(43) Veröffentlichungstag der Anmeldung:
**17.02.2021   Patentblatt 2021/07**

(73) Patentinhaber: **Imedos Systems GmbH**
**07751 Jena (DE)**

(72) Erfinder:
• **VILSER, Walthard**
**07407 Rudolstadt (DE)**
• **KRAUSS, Benedikt**
**07745 Jena (DE)**
• **RIEMER, Thomas**
**07751 Jena (DE)**

(74) Vertreter: **Gleim Petri Patent- und Rechtsanwaltspartnerschaft mbB**
**Neugasse 13**
**07743 Jena (DE)**

(56) Entgegenhaltungen:
**WO-A2-2006/091811**

• **EDGAR NAGEL ET AL: "Autoregulative behavior of retinal arteries and veins during changes of perfusion pressure: a clinical study", GRAEFE'S ARCHIVE FOR CLINICAL AND EXPERIMENTAL OPHTHALMOLOGY., Bd. 242, Nr. 1, 1. Januar 2004 (2004-01-01), Seiten 13-17, XP055547399, DE ISSN: 0721-832X, DOI: 10.1007/s00417-003-0663-3 in der Anmeldung erwähnt**
• **LOVASIK J V ET AL: "A novel noninvasive videographic method for quantifying changes in the chromaticity of the optic nerve head with changes in the intraocular pressure, pulsatile choroidal blood flow and visual neural function in humans", SURVEY OF OPHTHALMOLOGY, SURVEY OF OPHTHALMOLOGY INC, XX, Bd. 38, 1. Mai 1994 (1994-05-01), Seiten S35-S51, XP023265495, ISSN: 0039-6257, DOI: 10.1016/0039-6257(94)90045-0 [gefunden am 1994-05-01]**
• **XIAOYUN JIANG ET AL: "The effect of age on the response of retinal capillary filling to changes in intraocular pressure measured by optical coherence tomography angiography", MICROVASCULAR RESEARCH., Bd. 115, 1. Januar 2018 (2018-01-01), Seiten 12-19, XP055548460, US ISSN: 0026-2862, DOI: 10.1016/j.mvr.2017.08.001**

**Beschreibung**

Die Erfindung betrifft eine Vorrichtung und ein Verfahren, mit denen im Auge eines Patienten durch künstliches Einbringen eines Stimulationsdruckes SD die metabolische Autoregulation des Netzhautgefäßsystems untersucht werden kann. Damit sind die Vorrichtung und das Verfahren, als Funktionsuntersuchung, für Einsatzgebiete zur kausalen Diagnostik von retinalen Durchblutungsstörungen, für die Verlaufsbeobachtung und das Monitoring therapeutischer Wirkungen, aber vor allem für die präventive Diagnostik des Glaukoms und anderer retinaler Durchblutungsstörungen geeignet.

Unter Autoregulation versteht man verschiedene autoregulative Mechanismen mit unterschiedlicher Funktion und Dynamik. Unter metabolischer Autoregulation soll verstanden werden, dass die retinalen Gefäße auf Mangelsituationen reagieren, wenn der Metabolismus der Netzhaut nicht mehr ausreichend mit Sauerstoff und Nährstoffen versorgt wird oder gestört ist. Das ist u. a. der Fall, wenn der retinale Perfusionsdruck rPP der Netzhaut abfällt. In diesem Fall hat die Autoregulation die Aufgabe, den Druckabfall zu kompensieren, indem sie Gefäße der Netzhaut weit stellt.

Verschiedene wissenschaftliche Ergebnisse und technische Vorschläge setzen Verfahren zur Blutfluss- oder Blutgeschwindigkeitsmessung ein oder auch zur Erkennung kapillarer Verschlüsse (Fluoreszenzangiografie, OCT-Angiografie) ein. Die bisher bekannten bildgebenden Systeme können aber nur morphologische Schäden und keine funktionellen Störungen darstellen. Damit weist der Stand der Technik der gegenwärtigen morphologischen Bildgebung erhebliche Nachteile auf und soll nachfolgend nicht weiter betrachtet werden.

Methoden zur Untersuchung funktioneller autoregulativer Mechanismen auf der Basis von Gefäßdurchmessern sind in der Literatur beschrieben. Die Untersuchung der metabolischen Autoregulation auf der Basis der Messung von Gefäßdurchmessern ist in den Arbeiten von Nagel et al. (Nagel, E.; Vilser, W.: "Autoregulative behavior of retinal arteries and veins during changes of perfusion pressure: a clinical study." Graefe's Arch Clin Exp Ophthalmol (2004) 242: 13-17) beschrieben, welche der erfindungsgemäßen Lösung am nächsten kommen. Über das Suction-Cup-Verfahren nach Ulrich wird der Intraokulardruck IOP künstlich auf einen konstanten Wert erhöht. Der dafür notwendige Stimulationsdruck SD (negative SD-Werte im Saugnapf) wird dann über eine Stimulationszeitdauer T (z. B. 90 s) konstant gehalten und danach auf Null entlastet. Gemessen wurden mit dem Retinal Vessel Analyzer der Firma Imedos Gefäßdurchmesser ausgewählter großer Netzhautarterien und -venen, gefäßsegmentweise entlang von ausgewählten Gefäßabschnitten über eine Gesamtzeit in drei Phasen. In der ersten Phase (Baseline-Phase BP) werden Gefäßdurchmesser der großen Netzhautgefäße unbeeinflusst durch Änderungen des IOP bzw. des rPP, also ohne Stimulation (Provokation), untersucht. In einer zweiten Phase (Stimulationsphase SP) wird der retinale Perfusionsdruck rPP gesenkt bzw. der Intraokulardruck IOP schnell um einen vorgegebenen Änderungs-Intraokulardruckwert DIOP, erhöht und für die Stimulationszeitdauer T konstant gehalten. In der dritten Phase (Nachphase NP) wird der $dIOP_s$ schnell auf Null entlastet. Die über die Gefäßabschnitte gemittelten Gefäßdurchmesser wurden über die Gesamtzeit als Gefäßdurchmessersignale D(t,x,y) aufgezeichnet. Die Gefäßdurchmessersignale D(t,x,y) zeigen im Patientenmittel in den Phasen SP und NP signifikante Gefäßreaktionen, die die metabolische Autoregulation beschreiben. Die Baseline-Phase BP dient der Ermittlung von Referenzwerten, auf die die Gefäßreaktionen prozentual normiert werden.

Ein wesentlicher Nachteil des vorgenannten Standes der Technik ist, dass die metabolische Gefäßreaktion der Kapillaren nicht untersucht werden kann. Da man davon ausgehen muss, dass die Gefäßbereiche der arteriellen, venösen und kapillaren Gefäße unterschiedliche Gefäßreaktionen aufweisen und in den Kapillaren ein bedeutender Teil des Stoffaustausches erfolgt, ist die Kenntnis der kapillaren metabolischen Autoregulation von besonderem Interesse, zumindest aber notwendig, um die metabolische Autoregulation insgesamt zu verstehen und klinisch einordnen zu können.

Weitere Nachteile der im vorgenannten Stand der Technik beschriebenen Untersuchung bestehen in der Wahl des Suction-Cup-Verfahrens nach Ulrich. Das Verfahren erhöht bereits beim Anbringen des Suction Cups an das Auge den IOP unkontrolliert, verursacht beim Patienten Läsionen mit Blutergüssen am Auge durch den Saugdruck und ist für den Patienten zwar ertragbar, aber unangenehm. Die Saugwirkung verformt zudem den Augapfel und führt bereits sehr frühzeitig zu Astigmatismus, der zu Messfehlern bei der Messung der Gefäßreaktionen führt. Im Weiteren streuen die Gefäßreaktionen sehr stark. Als wesentliche Ursachen sind Tonografieeffekte und die verwendeten unsicheren Beziehungen zwischen Saugdruck und Änderung des Intraokulardruckes dIOP zu nennen, die als mittlerer Zusammenhang aus vielen verschiedenen Augen ermittelt wurden und beim Suction-Cup-Verfahren zur Berechnung der IOP-Werte für die Untersuchungen zum Einsatz kommen. Zusätzliche Tonometermessungen zeigten, dass der Tonografieeffekt vernachlässigbar gegen die große Unsicherheit der Eichbeziehung des Gerätes OODG nach Ulrich zwischen dIOP und Saugdruck ist.

Es ist die Aufgabe der Erfindung, ein Verfahren zu finden, mit dem parallel zu den Gefäßreaktionen der großen Gefäße die kapillaren metabolischen Gefäßreaktionen untersucht werden können. Darüber hinaus sollen vorteilhaft die Reproduzierbarkeit und der individuelle Aussagewert der Untersuchungsergebnisse deutlich verbessert werden. Vorteilhaft soll die Untersuchung für den Patienten angenehmer gestaltet werden.

Es ist auch die Aufgabe der Erfindung, eine zur Durchführung des Verfahrens geeignete Vorrichtung zu schaffen.

Das Wesen der Erfindung besteht darin, parallel zur Messung der Gefäßdurchmesser der großen Netzhautgefäße die Kapillargefäße durch spektral normierte Quotientensignale oder andere die Kapillaren beschreibende lokale Signale, wie z. B. die bewegende Blutzellendichte (auch als Kapillardichte bezeichnet), den kapillaren Fluss oder die kapillare Geschwindigkeit oder deren Plasma- oder Blutzellenbewegung, zu erfassen und damit deren Gefäßreaktionen aufzuzeichnen.

Als eine wesentliche Unsicherheit und Fehlerquelle der klinischen Aussage zur Untersuchung der Autoregulation, wie sie mit dem zuvor beschriebenen Verfahren getroffen wurde, wurde erkannt, dass für die untersuchungsbedingte Reduzierung des Perfusionsdruckes rPP als Ausgangswert nur der Ruhe-Intraokulardruck $IOP_0$, unbeachtet des retinalen Venendrucks außerhalb des Augapfels RVP, Berücksichtigung fand.

Es ist erfindungswesentlich, dass der Einfluss eines erhöhten retinalen Venendruckes außerhalb des Augapfels RVP auf die Untersuchung der Gefäßreaktion und Autoregulation ausgeschaltet wird bzw. die Untersuchung bezogen auf einen aktuellen individuellen retinalen Perfusionsdruck rPP standardisiert wird.

Vorteilhaft wird auch die starke Streuung des Zusammenhanges zwischen dem IOP und dem Stimulationsdruck SD durch direkte Messung des IOP oder einen individuell für das betreffende Auge ermittelten Zusammenhang zwischen dem IOP und dem SD erheblich eingeschränkt. Damit wird der über viele Augen bestimmte mittlere und stark streuende Zusammenhang zwischen dem IOP und dem SD, wie er im vorgenannten Stand der Technik benutzt wird, gegen eine genaue individuell ermittelte Beziehung ersetzt.

Der retinale Perfusionsdruck rPP berechnet sich aus der Differenz des retinalen arteriellen Blutdruckes rPa und des innerhalb des Augapfels herrschenden retinalen venösen Blutdruckes rPv. Dieser innerhalb des Augapfels herrschende retinale venöse Blutdruck rPv entspricht gewöhnlich bei gesunden Augen dem natürlichen Intraokulardruck IOP (gleich Ruhe-Intraokulardruck $IOP_0$). Der $IOP_0$ ist dann größer als der außerhalb des Auges herrschende retinale Venendruck RVP. In diesem Fall sieht man auf der Papille (Sehnervenkopf) einen sogenannten spontanen Venenkollaps. Der retinale Perfusionsdruck rPP berechnet sich dann:

$$rPP = rPa - rPv \quad \text{mit } IOP_0 > RVP \quad \text{folgt } rPv = IOP \quad \text{und} \quad rPP = rPa - IOP$$

Fehlt der Venenkollaps, ist dies ein Hinweis darauf, das der RVP größer als der $IOP_0$ ist, und der RVP bestimmt den retinalen Perfusionsdruck rPP dann nach folgender Formel:

$$rPP = rPa - rPv \quad \text{mit } RVP > IOP_0 \quad \text{folgt } rPv = RVP \quad \text{und} \quad rPP = rPa - RVP$$

Folglich wird der retinale Perfusionsdruck rPP nur dann vom $IOP_0$ des Auges bestimmt, wenn dieser größer als der retinale Venendruck außerhalb des Augapfels RVP ist. Im pathologischen Fall, insbesondere beim Glaukom, ist aber häufig der RVP höher als der $IOP_0$, und damit bestimmt der RVP den retinalen Perfusionsdruck rPP, wie aus den angegebenen Formeln zu sehen ist.

Basierend auf diesen Erkenntnissen wird die metabolische Autoregulation während einer standardisierten Reduzierung des retinalen Perfusionsdruckes rPP untersucht, die auf den tatsächlichen rPP bezogen wird und von einem IOP-Wert ausgeht, bei dem ein spontaner Venenkollaps festgestellt wird.

Wird der spontane Venenkollaps bereits ohne eine Erhöhung des IOP festgestellt, dann wird der Ausgangs-IOP-Wert, der bei einer druckfreien Anlage eines Druckapplikators der Ruhe-Intraokulardruckwert $IOP_0$ ist, um einen vorgegebenen Änderungs-Intraokulardruckwert DIOP, auf einen Stimulations-Intraokulardruckwert $IOP_S$ angehoben. Der retinale Stimulations-Perfusionsdruck $rPP_s$ beträgt dann während der Stimulationsphase SP:

$$rPP_s = rPP - dIOP_s = rPa - IOP_0 - dIOP_s \quad \text{(bei } IOP_0 > RVP) \quad \text{(Formel 1)}$$

Unter diesen Bedingungen wurden auch die Untersuchungen des Standes der Technik durchgeführt.

Erfindungsgemäß ergibt sich unter Berücksichtigung eines über den $IOP_0$ erhöhten retinalen Venendruckes außerhalb des Augapfels $RVP > IOP_0$ für $rPP_s$ die nachfolgende Berechnungsformel:

$$rPP_s = rPP - dIOP_s = rPa - RVP - dIOP_s$$

$$= rPa - IOP_0 - dIOP_{RVP} - dIOP_s \quad \text{für } (IOP_0 < RVP) \quad \text{(Formel 2)}$$

wobei der Wert $dIOP_{RVP}$ der Wert ist, um den der $IOP_0$ erhöht werden muss, um den spontanen Venenkollaps auszulösen und den RVP zu messen.

Die Auswirkungen der Vernachlässigung eines RVP, wenn dieser größer dem Ruhe-Intraokulardruck $IOP_0$ ist, ist in Formel 2 zu erkennen. Um eine gleiche Reduzierung des retinalen Perfusionsdruckes rPP um den vorgegebenen $dIOP_s$ zur erhalten, muss bei erhöhtem RVP ($RVP > IOP_0$) der IOP nicht um $dIOP_s$ gegenüber dem $IOP_0$ erhöht werden, sondern gegenüber $DIOP_s + dIOP_{RVP}$. Der Wert $dIOP_{RVP}$ ist genau der Wert, um den RVP größer ist als der $IOP_0$.

Klinisch bedeutet dies, dass im Falle des Standes der Technik die Erhöhung des IOP um DIOP, nach Formel 1 richtig ist für $IOP_0 > RVP$, im anderen Fall aber falsch ist, weil im Extremfall der retinale Perfusionsdruck rPP gar nicht gesenkt wurde, womit in diesem Fall auch keine Gefäßreaktion zu erwarten ist. Für den Stand der Technik kann daher davon ausgegangen werden, das bei den Studien der rPP nicht standardisiert, sondern sehr unterschiedlich reduziert oder sogar teilweise gar nicht reduziert wurde. Daraus ergibt sich eine große Streuung der Gefäßreaktionen, je nach Zusammensetzung der Patientengruppe und deren RVP- bzw. IOP-Werten.

Erfindungsgemäß wird der RVP bei der Berechnung des dem $IOP_S$ zugehörigen Stimulationsdruckwertes $SD_s$ berücksichtigt und schaltet somit einen gerade in pathologischen Fällen erheblichen Fehlereinfluss aus.

Die Aufgabe der Erfindung wird für eine Vorrichtung zur Untersuchung der metabolischen Autoregulation der Netzhautgefäße eines Auges eines Patienten, enthaltend eine auf das Auge wirkende Einheit zur Erzeugung und Applikation eines Stimulationsdruckes und eine Bildgebungseinheit, dadurch gelöst, dass die Bildgebungseinheit eine modifizierte Netzhautkamera mit digitalem Bildsensor ist, die von der Netzhaut eine Videosequenz von Bildern, denen jeweils zwei Farbkanäle zugeordnet sind, erzeugt, und eine Einheit zur Bildung von spektral normierten Quotientensignalen vorhanden ist, die aus Intensitätssignalen der Farbkanäle Quotientensignale ableitet, aus denen auf die Gefäßreaktion und damit die metabolische Autoregulation der Kapillaren der Netzhautgefäße geschlossen werden kann. Alternativ wird die Aufgabe für eine Vorrichtung dadurch gelöst, dass die Bildgebungseinheit auf Basis der Laser-Scanning-Technik oder der optischen Kohärenztomographie beruht, die von der Netzhaut eine Videosequenz von Bildern erzeugt, aus denen Signale ableitbar sind, zur Beschreibung von lokalen Gefäßdurchmessern, von lokalen Blutgeschwindigkeiten, von lokalen Blutflüssen oder von lokalen Kapillardichten der Kapillaren oder / und der großen Gefäße. Ferner wird die Aufgabe für eine Vorrichtung dadurch gelöst, dass ein Tonometer vorhanden ist, um einen Intraokulardruck IOP im Auge, der sich in Abhängigkeit von einem von der Einheit zur Erzeugung und Applikation eines Stimulationsdruckes applizierten Stimulationsdruck ändert, zu messen, und dass die Einheit zur Erzeugung und Applikation eines Stimulationsdruckes einen Sensor zur Messung des Stimulationsdruckes enthält, um jeweils einem gemessenen Intraokulardruckwert IOP und jedem Bild der Videosequenz einen Stimulationsdruckwert zuordnen zu können.

Die Aufgabe der Erfindung kann auf der Basis verschiedener abbildender Einheiten gelöst werden, wobei jeweils Signale von den großen Gefäßen und Kapillaren gebildet werden, die den lokalen Gefäßdurchmesser oder die Kapillardichte, Blutzellen- oder Blutplasmageschwindigkeit oder den Blutfluss oder sonstig die Gefäßperfusion beschreiben.

Vorteilhaft enthält die Einheit zur Erzeugung und Applikation eines Stimulationsdruckes einen Druckapplikator, der am Kopf des Patienten ortsfest zum Auge außerhalb der Hornhaut und eines Lichtweges der Bildgebungseinheit druckfrei flächig an das Auge anlegbar ist.

Es ist ferner von Vorteil, wenn die Bildgebungseinheit eine spektral modifizierte Netzhautkamera ist, die in ihrem Beleuchtungsstrahlengang einen doppelbandigen Bandpassfilter mit einem Spektralbereich im roten und einem Spektralbereich im grünen Licht aufweist.

Die Aufgabe der Erfindung wird ferner für ein Verfahren zur Untersuchung der metabolischen Autoregulation der Netzhautgefäße eines Auges eines Patienten, bei dem eine Videosequenz von Bildern der Netzhautgefäße während einer das Auge unbeeinflussenden Baseline-Phase, einer Stimulationsphase, in der ein Intraokulardruck IOP durch Anlegen und Erhöhen eines auf das Auge wirkenden Stimulationsdruckes SD um einen vorgegebenen Änderungs-Intraokulardruckwert DIOP, erhöht und über eine Stimulationszeitdauer auf einem Stimulations-Intraokulardruckwert $IOP_S$ gehalten wird, und einer das Auge unbeeinflussenden Nachphase aufgezeichnet wird und bei dem aus den Bildern der Videosequenz Signale abgeleitet werden, die die lokale Perfusion von Gefäßen (Gefäßperfusion) beschreiben, dadurch gelöst, dass die Erhöhung um den vorgegebenen Änderungs-Intraokulardruckwert DIOP, ausgehend von einem gemessenen Ruhe-Intraokulardruckwert $IOP_0$ erfolgt, wenn während der Baseline-Phase Messkriterien für einen spontanen Venenkollaps am Sehnervenkopf auf der Netzhaut festgestellt werden, dass sie ausgehend von einem erhöhten Intraokulardruckwert $IOP_{RVP}$ erfolgt, wenn während einer Erhöhung des Intraokulardrucks IOP bei Erreichen des erhöhten Intraokulardruckwertes $IOP_{RVP}$ Messkriterien für einen spontanen Venenkollaps am Sehnervenkopf auf der Netzhaut festgestellt werden, und dass sie ausgehend von dem gemessenen Ruhe-Intraokulardruckwert $IOP_0$ erfolgt, wenn kein spontaner Venenkollaps, auch nicht während der Erhöhung des Intraokulardrucks IOP am Sehnervenkopf auf der Netzhaut festgestellt wird.

Vorzugsweise sind die für die Gefäßreaktion der Netzhautgefäße für arterielle und venöse Gefäße beschreibenden Signale Gefäßdurchmessersignale und die für Kapillargefäße beschreibenden Signale sind spektral normierte Quotientensignale, die parallel erfasst und aufgezeichnet werden.

Bevorzugt wird nach der Messung des Ruhe-Intraokulardruckwertes $IOP_0$ unter Einwirkung eines Stimulationsdruckes SD wenigstens ein zweiter Intraokulardruckwert IOP gemessen und zwischen den Intraokulardruckwerten IOP und den jeweils zugehörigen Stimulationsdruckwerten SD wird ein für das betreffende Auge individueller Zusammenhang ermittelt, um den Stimulations-Intraokulardruckwert $IOP_S$ über einen individuell zugehörigen Stimulationsdruckwert $SD_s$ einzustellen.

Darüber hinaus ist es vorteilhaft, wenn mit dem Beginn der Stimulationsphase der Stimulationsdruck SD mit mindestens 1 mmHg pro Sekunde erhöht wird, um den Ruhe-Intraokulardruckwert $IOP_0$ schnell um den vorgegebenen Änderungs-Intraokulardruckwert DIOP, auf den Stimulations-Intraokulardruckwert $IOP_S$ zu erhöhen.

Vorzugsweise wird nach dem Ablauf der Stimulationszeitdauer die Stimulationsphase beendet und der Stimulationsdruck SD schlagartig auf Null zurückgefahren.

Nachfolgend wird die Erfindung an einem Ausführungsbeispiel für eine Vorrichtung und für ein Verfahren unter Verwendung einer modifizierten konventionellen Netzhautkamera anhand von Zeichnungen näher erläutert. Hierzu zeigen:

Fig. 1: ein Blockschaltbild für eine erfindungsgemäße Vorrichtung,

Fig. 2: eine Ausführung einer Einheit zur Erzeugung und Applikation eines Stimulationsdruckes,

Fig. 3a: einen zeitlichen Ablauf für die Untersuchung, wenn sich der retinale Perfusionsdruck rPP aus dem Ruhe-Intraokulardruckwert $IOP_0$ ergibt,

Fig. 3b: einen zeitlichen Ablauf für die Untersuchung, wenn sich der retinale Perfusionsdruck rPP aus einem erhöhten Intraokulardruckwert $IOP_{RVP}$ ergibt, der einem RVP größer dem Ruhe-Intraokulardruck $IOP_0$ entspricht,

Fig. 4a: einen zeitlichen Verlauf eines Gefäßdurchmessersignals für eine Arterie und eine Vene während der drei Untersuchungsphasen und

Fig. 4b: einen zeitlichen Verlauf eines Quotientensignals während der drei Untersuchungsphasen.

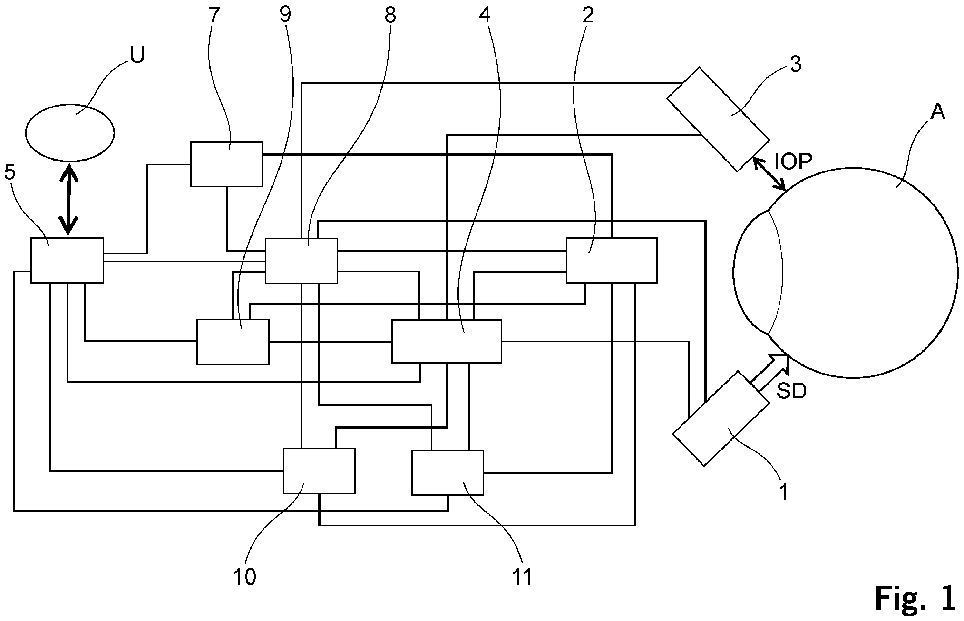

Eine erfindungsgemäße Vorrichtung enthält, wie in Fig. 1 in einem Blockschaltbild dargestellt, mindestens eine Einheit zur Erzeugung und Applikation eines Stimulationsdruckes 1, eine Bildgebungseinheit 2 mit einem digitalen Bildsensor, ein Tonometer 3, eine Rechen- und Steuereinheit 4, eine Ein- und Ausgabeeinheit 5, eine Ergebnisbildspeichereinheit 7, eine Daten- und Bildauswerteeinheit 8, eine Signalanalyseeinheit 9, eine Einheit zur Bildung von spektral normierten Quotientensignalen 10 und eine Einheit zur Bildung von Gefäßdurchmessersignalen 11.

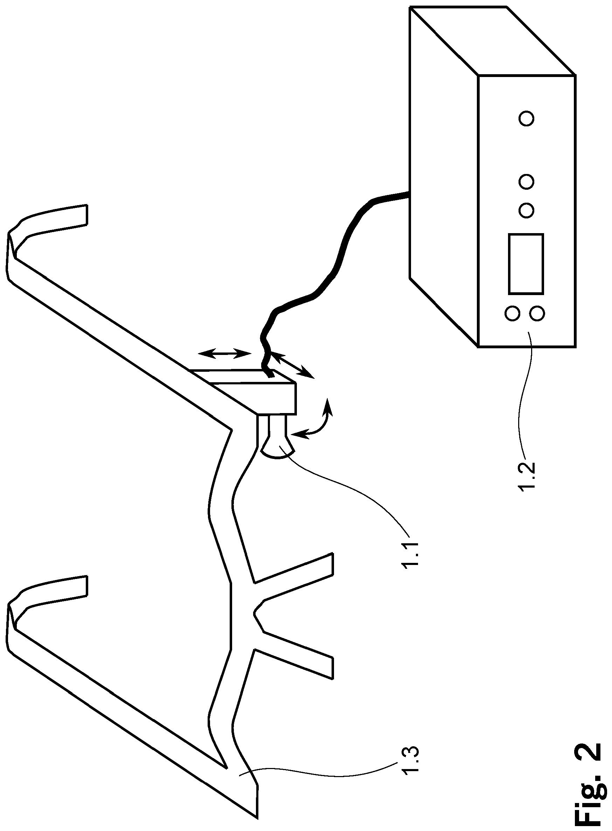

Die Einheit zur Erzeugung und Applikation eines Stimulationsdruckes 1 weist, wie in Fig. 2 schematisch angedeutet, eine Druckerzeugungseinheit 1.2, eine Halterung 1.3 und einen Druckapplikator 1.1 auf, der über die vorzugsweise brillenähnliche Halterung 1.3 jeweils seitlich (temporal) am rechten und linken Auge A des Patienten fixiert ist. Der Druckapplikator 1.1 kann druckfrei am zu untersuchenden Auge A des Patienten flächig angelegt werden.

Der Druckapplikator 1.1 dient dem Einbringen eines Stimulationsdruckes SD auf das zu untersuchende Auge A des Patienten und ist bevorzugt ein kleiner pneumatischer Ballon, könnte aber z. B. auch ein Stempel, ein Saugnapf oder ein hydraulisches System sein.

Die Ausführung des Druckapplikators 1.1 als kleiner pneumatischer Ballon birgt im Gegensatz zu Druckapplikatoren, wie sie aus Vorrichtungen oder Verfahren des Standes der Technik bekannt sind, eine Reihe von Vorteilen. So ist zum Beispiel das Verletzungsrisiko durch scharfe Kanten, welche an den Rändern von Druckapplikatoren 1.1 aus Metallen, Kunststoffen, Keramiken oder anderen festen Materialien hergestellt werden, deutlich geringer. Darüber hinaus ist die weiche Oberfläche des Ballons während der Untersuchung deutlich angenehmer für den Patienten. Zusätzlich werden durch die in alle Richtungen gleichmäßig ausgeführte Ausbreitung des Ballons Querkräfte vermieden, welche zur Verfälschung der Messergebnisse führen können.

Mit dem Druckapplikator 1.1 steht die Druckerzeugungseinheit 1.2 in Verbindung, mit der der Stimulationsdruck SD erzeugt, erhöht, abgesenkt und konstant gehalten werden kann.

Zur gesteuerten Erzeugung des Stimulationsdruckes SD ist die Einheit zur Erzeugung und Applikation eines Stimulationsdruckes 1 mit der Rechen- und Steuereinheit 4 verbunden. Je nach gewähltem Druckapplikator 1.1 kann die Einheit zur Erzeugung und Applikation eines Stimulationsdruckes 1 als Druckerzeugungseinheit 1.2 z. B. eine Pumpe, ein System aus einem Pneumatikzylinder und einem Kolben oder / und eine Steuerelektronik für Linearantriebe enthalten. Vorteilhaft ist die Druckerzeugungseinheit 1.2 ein pneumatisches System aus einem Pneumatikzylinder und einem Kolben, welcher mit Hilfe eines Linearantriebes in dem Pneumatikzylinder verschoben werden kann. Durch die Verschiebung des Kolbens wird die im pneumatischen System enthaltene Luft komprimiert oder dilatiert, wodurch es zu einem Druckanstieg oder Druckabfall im Druckapplikator 1.1 kommt. Die Druckerzeugungseinheit 1.2 kann vorteilhaft Komponenten zur definierten Einstellung des Anstieges oder Abfalls des Stimulationsdruckes SD enthalten. Mögliche Ausführungen sind hierfür z. B. Systeme aus verschiedenen Drossel- und Magnetventilen oder eine geeignete Steuerelektronik, welche unterschiedliche Geschwindigkeiten bei der Verstellung eines Linearantriebs ermöglicht.

Die Druckerzeugungseinheit 1.2 weist einen Sensor zur Messung des Stimulationsdruckes SD auf. Hierfür können je nach Ausführung z. B. Drucksensoren, Kraftsensoren oder Abstandssensoren eingesetzt werden.

Die Druckerzeugungseinheit 1.2 weist vorteilhaft auch eine Komponente auf, welche ein schlagartiges Absenken des Stimulationsdruckes SD ermöglicht. Hierfür können zum Beispiel ein oder mehrere Magnetventile verwendet werden, mit denen das System im Notfall schlagartig entlüftet wird.

Die Halterung 1.3 dient einer direkten Kopplung des Druckapplikators 1.1 am Kopf des Patienten und kann z. B. eine Brille, ein Stirnband oder ein Bügel sein, welche(r) über den Kopf des Patienten gelegt wird. Vorzugsweise wird die Halterung 1.3 in Form einer Brille umgesetzt. Zur Verbesserung der Kopplung des Druckapplikators 1.1 am Kopf des Patienten ist vorteilhaft an der Halterung 1.3 eine weitere Komponente, wie ein Brillenband, ein Gummiband oder eine mechanisch verstellbare Fixiermöglichkeit, vorgesehen.

Zur Realisierung einer individuell anpassbaren Positionierung des Druckapplikators 1.1, insbesondere einer Verstellbarkeit der Richtung, aus der der Druckapplikator 1.1 an das Auge A des Patienten herangeführt wird, ist der Druckapplikator 1.1 am Auge A des Patienten an der Halterung 1.3 bevorzugt über eine Höhenverstellung, eine Entfernungsverstellung und eine Winkelverstellung individuell verstellbar angebracht.

Der optische Zugang (Lichtweg) zur Netzhaut für die Bildgebungseinheit 2 darf durch keine der in der Einheit zur Erzeugung und Applikation eines Stimulationsdruckes 1 enthaltenen Komponenten beeinträchtigt und / oder versperrt werden.

Das Tonometer 3 ist hier vorteilhaft ein modifiziertes Rebound-Tonometer. Es ist über Signalwege mit der Rechen- und Steuereinheit 4 und der Daten- und Bildauswerteeinheit 8 verbunden. Es ist in die Vorrichtung integriert und vollautomatisch von dieser gesteuert. Über die Verbindung zur Rechen- und Steuereinheit 4 werden beim Erreichen zuvor definierter Messkriterien automatisch durchgeführte Messungen des Intraokulardrucks IOP ausgelöst. Die ermittelten Intraokulardruckwerte IOP werden an die Rechen- und Steuereinheit 4 übermittelt und dort zur weiteren Verarbeitung auf ein Zeitsignal synchronisiert. Die auf das Zeitsignal synchronisierten Intraokulardruckwerte IOP werden zur Speicherung und weiteren Verarbeitung an die Daten- und Bildauswerteeinheit 8 gesendet.

Die Rechen- und Steuereinheit 4 ist mit der Einheit zur Erzeugung und Applikation eines Stimulationsdruckes 1, der Bildgebungseinheit 2, dem Tonometer 3 und der Ein- und Ausgabeeinheit 5, der Daten- und Bildauswerteeinheit 8, der Signalanalyseeinheit 9, der Einheit zur Bildung von spektral normierten Quotientensignalen 10 und der Einheit zur Bildung von Gefäßdurchmessersignalen 11 signaltechnisch verbunden. Die Funktion der Daten- und Bildauswerteeinheit 8, die mit der Rechen- und Steuereinheit 4, der Ergebnisbildspeichereinheit 7, der Ein- und Ausgabeeinheit 5, der Bildgebungseinheit 2, der Einheit zur Erzeugung und Applikation eines Stimulationsdruckes 1, der Signalanalyseeinheit 9, der Einheit zur Bildung von spektral normierten Quotientensignalen 10 und der Einheit zur Bildung von Gefäßdurchmessersignalen 11 über Signalleitungen verbunden ist, wird anhand der Beschreibung des Verfahrens erläutert.

Die Ergebnisbildspeichereinheit 7, die mit der Bildgebungseinheit 2, der Ein- und Ausgabeeinheit 5 und der Daten- und Bildauswerteeinheit 8 signaltechnisch verbunden ist, dient der Speicherung bzw. Zwischenspeicherung von die Gefäßreaktionen widerspiegelnden Messwerten oder Signalen in Ergebnisbildern wie z. B. dem Mappingbild.

Signale, die der Signalanalyseeinheit 9 zur Analyse übergeben werden, sind z. B. Gefäßdurchmessersignale D(t,x,y). Sie werden als eine zeit- und ortsabhängige Gefäßdurchmesseränderung einzelner Gefäßsegmente oder der für einen aus mehreren Gefäßsegmenten gebildeten Gefäßabschnitt gemittelte mittlere Gefäßdurchmesser in der Einheit zur Bildung von Gefäßdurchmessersignalen 11, gebildet. Andere solche Signale über eine definierte Fläche eines Pixels oder einer Gruppe von Pixeln (Messort) können z. B. ein gemitteltes unnormiertes Helligkeitssignal, oder / und ein gemitteltes spektral normiertes Quotientensignal Q(t,x,y) sein.

Die Ein- und Ausgabeeinheit 5 dient zur Eingabe von Daten und Steuerbefehlen durch einen Untersucher U und zur Darstellung und Ausgabe der jeweiligen Untersuchungsergebnisse. Während der Untersuchung können die Stimulationsdruckwerte SD und Videosequenzen auf dem Monitor online dargestellt werden. Der Untersucher U kann über einen zur Eingabe- und Ausgabeeinheit 5 gehörigen Monitor die Videosequenzen beobachten und die fertigen Untersuchungsergebnisse präsentiert bekommen. Die Videosequenzen werden vorzugsweise dem Untersucher U gemeinsam mit Messergebnissen zur Verfolgung und Kontrolle der Einstellung der Bildgebungseinheit 2 während des Untersuchungsvorganges präsentiert.

Die Bildgebungseinheit 2 ist z. B. eine spektral modifizierte Netzhautkamera, mit der von der Netzhaut Videosequenzen von Bildern mit zwei Farbkanälen erzeugt werden.

Vorteilhaft weist die Bildgebungseinheit 2 in ihrem Beleuchtungsstrahlengang einen doppelbandigen Bandpassfilter auf, z. B. mit einem Spektralbereich im roten und einem Spektralbereich im grünen Licht, und die spektralen Anteile werden auf getrennte Bereiche oder verschiedene Chips eines digitalen Bildsensors geleitet, so dass ein rotes und ein grünes Bild entstehen, die jeweils als einer der zwei Farbkanäle eines Bildes zu verstehen sind.

Alternativ kann die Bildgebungseinheit 2 einen digitaler Bildsensor aufweisen, bei dem jedes Pixel aus mindestens zwei Subpixeln unterschiedlicher spektraler Empfindlichkeit, z. B. rot und grün, gebildet ist. Jeweils über eines der Subpixel eines Pixels des digitalen Bildsensors wird einer von zwei Farbkanälen der Bilder der Videosequenz erzeugt. Die Einheit zur Bildung von spektral normierten Quotientensignalen 10 bildet aus den roten und grünen Farbintensitätssignalen der Farbkanäle der Bilder bzw. der Subpixel, die gemeinsam ein Pixel bilden, pixelweise Quotienten, wobei die Pixel in den aufeinanderfolgenden Bildern dem gleichen Messort entsprechen müssen. Damit entsteht ein spektral normiertes Quotientenbild, bei dem beleuchtungsseitige Unterschiede durch spektrale Normierung beseitigt sind. Das rote rückgestreute Licht, das Blut im Wesentlichen durchstrahlt, dient dabei als Referenzwellenlänge, wobei Licht im grünen Licht stark von Blut absorbiert wird und das Blutvolumen in einem Netzhautbereich widerspiegelt. Das spektral normierte Quotientensignal $Q(t,x,y)$ beschreibt somit beleuchtungsunabhängig das Blutvolumen in einem Kapillarbereich. Die dadurch entstehende Quotientenbildsequenz von der Netzhaut wird in der Einheit zur Bildung von spektral normierten Quotientensignalen 10 gespeichert und dann entsprechend der Verfahrensschritte an die Signalanalyseeinheit 9 als spektral normiertes Quotientensignal $Q(t,x,y)$ geleitet. Als Bildgebungseinheit 2 kann auch jede andere bildgebende Vorrichtung eingesetzt werden, die Bilder mit mehreren Farbkanälen erzeugen kann.

Für die Erfindung ist es unerheblich, wie und über welche Wellenlängen die spektrale Normierung erfolgt, sofern beleuchtungsunabhängige Signale entstehen, die das Blutvolumen in einem Gewebevolumen beschreiben.

Die Signalanalyseeinheit 9 ist mit der Ein- und Ausgabeeinheit 5, der Daten- und Bildauswerteeinheit 8, der Rechen- und Steuereinheit 4 sowie mit der Bildgebungseinheit 2 verbunden. Ihre Funktionsweise wird später anhand der Beschreibung eines Ausführungsbeispiels des erfindungsgemäßen Verfahrens erläutert.

Die Einheit zur Bildung von spektral normierten Quotientensignalen 10 ist mit der Ein- und Ausgabeeinheit 5, der Daten- und Bildauswerteeinheit 8 sowie mit der Bildgebungseinheit 2 verbunden. Die Einheit zur Bildung von spektral normierten Quotientensignalen 10 dient, wie bereits erläutert, der Ausschaltung der Abhängigkeit der Beleuchtungsintensität auf die für die Untersuchung gebildeten Signale.

Die Einheit zur Bildung von Gefäßdurchmessersignalen 11 ist mit der Ein- und Ausgabeeinheit 5, der Daten- und Bildauswerteeinheit 8 sowie mit der Bildgebungseinheit 2 verbunden. Die Einheit zur Bildung von Gefäßdurchmessersignalen 11 bestimmt Gefäßdurchmesser in mindestens einem selektierten Gefäß segmentweise entlang der Gefäßabschnitte sowie von Bild zu Bild im vorzugsweise grünen Farbkanal der Videosequenz oder wahlweise im Quotientenbild. Aus der zeitlichen Folge der Gefäßdurchmesser der einzelnen Gefäßsegmente werden dann Gefäßdurchmessersignale $D(t,x,y)$ gebildet, die der Signalanalyseeinheit 9 zugeleitet werden.

Die Vorrichtung muss nicht zwangsweise sowohl eine Einheit zur Bildung von spektral normierten Quotientensignalen 10 als auch eine Einheit zur Bildung von Gefäßdurchmessersignalen 11 besitzen und muss auch nicht zwangsweise spektral normierte Quotientenbilder und daraus abgeleitete spektral normierte Quotientensignale $Q(t,x,y)$ bilden. Die vorgestellten Vorschläge dieses Ausführungsbeispiels stellen vorteilhafte Ausführungen dar.

Zur Durchführung eines erfindungsgemäßen Verfahrens wurden bereits in der Beschreibung des Wesens der Erfindung und in der Beschreibung der Funktionsweise der Vorrichtung Ausführungen gemacht, die der nachfolgenden Beschreibung des Verfahrensablaufes zuzurechnen sind, auch wenn nicht in jedem Fall explizit darauf Bezug genommen wird.

Zur Vorbereitung der Untersuchung bringt der Untersucher U den Druckapplikator 1.1 am Kopf des Patienten derart an, dass der Druckappliator 1.1, ohne Druck auszuüben, das Auge A des Patienten im temporalen Lidwinkel leicht berührt.

Dann stellt er das Tonometer 3 derart auf das Auge A ein, dass parallel zur Bildgebung durch die modifizierte Netzhautkamera automatische Tonometermessungen möglich sind und auf dem Monitor der Ein- und Ausgabeeinheit 5 ein auswertbares Bild von der Netzhaut mit dem Sehnervenkopf (Augenhintergrund) angeboten werden kann.

Die von der Bildgebungseinheit 2 gelieferten Videosequenzen von Bildern werden automatisch auf ausreichende Bildqualität geprüft. Ist die Bildqualität nicht ausreichend, wird der Untersucher U über die Ein- und Ausgabeeinheit 5 aufgefordert, durch Einstellung der die Bildgebungseinheit 2 bildenden modifizierten Netzhautkamera die Bildqualität zu korrigieren.

Dann startet der Untersucher U den Untersuchungsvorgang mit einer ersten Phase, der sogenannten Baseline-Phase BP.

Es erfolgt eine automatische Messung des Intraokulardrucks IOP und der gemessene Intraokulardruckwert IOP, gleich dem Ruhe-Intraokulardruckwert $IOP_0$, wird als Ausgangswert gespeichert.

Anschließend oder während dessen wird mit der modifizierten Netzhautkamera über die Dauer von drei Phasen der Untersuchung eine Videosequenz von Bildern, denen zwei Farbkanäle zugeordnet sind, erzeugt, aus denen neben Gefäßdurchmessersignalen $D(t,x,y)$ insbesondere spektral normierte Quotientensignale $Q(t,x,y)$ zur Bewertung der metabolischen Autoregulation von Kapillaren gebildet werden.

In der Baseline-Phase BP werden alle Signale noch ohne Erhöhung des Stimulationsdruckes SD gebildet. Sie dienen bei einer späteren Auswertung der Signale über die anschließende Stimulationsphase SP und die Nachphase NP als Referenzwerte.

Zuvor werden über die Daten- und Bildauswerteeinheit 8 die von der Bildgebungseinheit 2 erzeugten Videosequenzen von Bildern der Netzhaut auf Bildverschiebungen bzw. Verdrehungen zwischen zeitlich aufeinanderfolgenden Bildern analysiert und derart korrigiert, dass eine bewegungskorrigierte Videosequenz erzeugt wird, bei der sich in den Bildern gleiche Netzhautpunkte überdecken. Die Bildung aller Signale erfolgt anhand dieser bewegungskorrigierten Videosequenz.

Zur Untersuchung der arteriellen und venösen Gefäße werden aus der bewegungskorrigierten Videosequenz Gefäßdurchmessersignale $D(t,x,y)$ gebildet. Dazu werden im Bereich der Papille (Sehnervenkopf) die arteriellen und venösen Gefäße selektiert und deren Messort gespeichert. Die Einheit zur Bildung von Gefäßdurchmessersignalen 11 greift dann auf die gespeicherten arteriellen und venösen Gefäße zu und bestimmt gefäßsegmentweise entlang der Gefäße sowie von Bild zu Bild Gefäßdurchmesser, deren Werte jeweils dem Messort auf der Netzhaut und der Zeit bzw. dem jeweiligen Bild zugeordnet und gespeichert werden, und bildet ein zeit- und ortsabhängiges Gefäßdurchmessersignal $D(t,x,y)$. Den Messorten können einzelne Pixel oder auch Pixelgruppen zugeordnet sein.

Die Daten- und Bildauswerteeinheit 8 bildet aus den Farbkanälen der Bilder der Videosequenz bzw. Intensitätswerten der zugeordneten Pixel bzw. Subpixel grüne Farbintensitätssignale und rote Farbintensitätssignale und bildet spektral normierte Quotientensignale $Q(t,x,y)$, deren zeitlicher Verlauf den Pixeln am Messort auf der Netzhaut, einem Zeitsignal, auf das auch alle Messergebnisse in Form von Messwerten oder Signalen synchronisiert werden, oder den Bildern zugeordnet wird.

Alle gebildeten Signale werden der Signalanalyseeinheit 9 zugeleitet und parallel als Zeitverläufe aufgezeichnet (gespeichert).

Die Rechen- und Steuereinheit 4 ordnet alle aktuellen SD-Werte einem Zeitsignal zu, das mit dem ersten START-Signal für die Untersuchung gleich NULL gesetzt wird und zu dem ab diesem Zeitpunkt auch alle originalen und abgeleiteten Videosequenzen, Bilder, Quotientenbilder und Signale synchronisiert bzw. zeitlich zugeordnet werden. Während der Baseline-Phase BP und der anschließenden Erhöhung des Intraokulardruckes IOP zu Beginn der Stimulationsphase SP überwacht die Signalanalyseeinheit 9 alle Signale bezüglich der nachfolgend definierten objektiven Messkriterien:

Die Signale auf dem selektierten Sehnervenkopf werden bezüglich des Auftretens eines spontanen Venenkollaps überwacht. Als objektives Messkriterium werden benutzt:

a) Die Durchmesser einzelner venöser Gefäßsegmente auf dem Sehnervenkopf beginnen deutlich stärker als in der Vergangenheit oder stärker als die meisten venösen Gefäßsegmente auf dem Sehnervenkopf zu pulsieren. Der Schwellenwertfaktor für die Zunahme der Pulsationsamplitude, bestimmend dafür, wann das Messkriterium eingetreten ist, wird mit 3 festgelegt, kann aber anhand experimenteller Untersuchungen auch anders eingestellt werden.

b) Die Signale, Quotientensignal $Q(t,x,y)$ und / oder rotes Farbintensitätssignal und / oder grünes Farbintensitätssignal, aus dem Bereich des Sehnervenkopfes steigen in ihrer Pulsamplitude deutlich gegenüber der Vergangenheit oder gegenüber den Signalen benachbarter Pixel an. Der Schwellenwertfaktor für die Zunahme der Pulsationsamplitude, bestimmend dafür, wann das Messkriterium eingetreten ist, wird mit 3 festgelegt, kann aber anhand experimenteller Untersuchungen anders oder zwischen den verschiedenen Signalen auch unterschiedlich eingestellt werden.

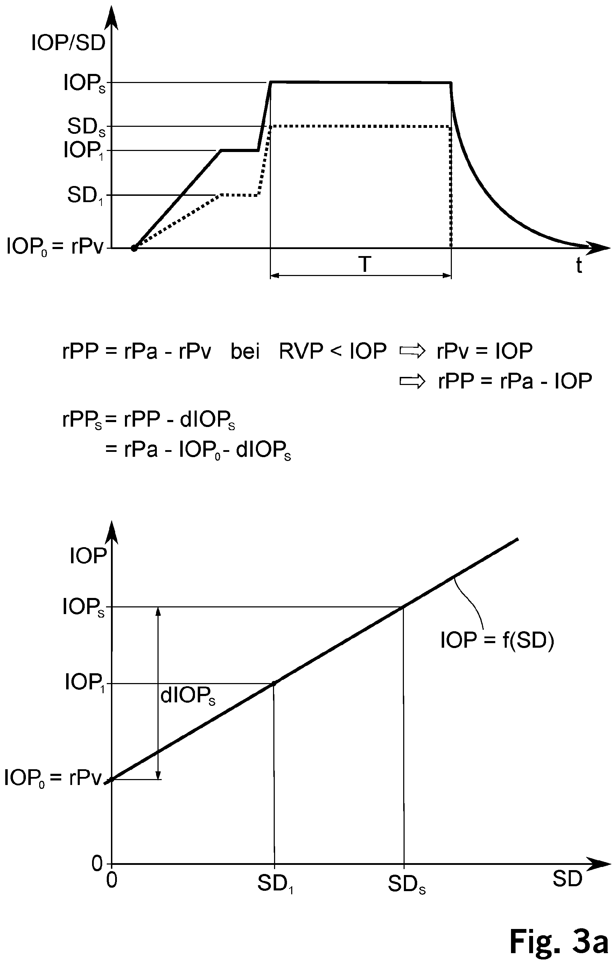

Erkennt die Signalanalyseeinheit 9 bereits während der Baseline-Phase BP, dass mindestens eines der Messkriterien für den Venenkollaps erfüllt ist, siehe hierzu **Fig. 3a,** wird der Ruhe-Intraokulardruckwert $IOP_0$ dem retinalen venösen Blutdruck innerhalb des Augapfels rPv gleichgesetzt. In diesem Fall bestimmt der Ruhe-Intraokulardruckwert $IOP_0$ den retinalen Perfusionsdruck rPP und die eingangsseitig aufgestellte Formel 1 findet Anwendung, wie auch im Stand der Technik. Ein gewünschter Stimulations-Intraokulardruckwert $IOP_S$ ergibt sich aus der Addition des Ruhe-Intraokulardruckwertes $IOP_0$ und eines vorgegebenen Änderungs-Intraokulardruckwertes $dIOP_s$ zur Stimulierung des Auges A.

$$IOP_s = IOP_0 + dIOP_s$$

In diesem Fall, aber auch wenn kein spontaner Venenkollaps erkannt wurde, wird die Stimulationsphase SP über die Rechen- und Steuereinheit 4 gestartet. Es wird ein START-Signal an die Einheit zur Erzeugung und Applikation eines Stimulationsdruckes 1 gegeben und das Ansteigen des Stimulationsdruckes SD im Druckapplikator 1.1 wird ausgelöst. Der Stimulationsdruck SD im Druckapplikator 1.1 soll mit mindestens 1 mmHg pro Sekunde ansteigen, um den Ruhe-Intraokulardruckwert $IOP_0$ schnell um den vorgegebenen Änderungs-Intraokulardruckwert $dIOP_s$ auf den Stimulations-Intraokulardruckwert $IOP_S$ zu erhöhen.

Während des Hochfahrens der Stimulationsdruckwerte SD wird mindestens eine weitere Intraokulardruckmessung durchgeführt, in **Fig. 3a** als IOP, angegeben, um die für das Auge A individuelle Abhängigkeit des verursachenden Stimulationsdruckes SD in Abhängigkeit vom erreichten Intraokulardruck IOP bzw. von dessen Änderung, SD = f(IOP), zu errechnen und darüber einen dem Stimulations-Intraokulardruckwert $IOP_S$ zugehörigen Stimulationsdruckwert $SD_S$ zu berechnen, der letztlich durch die Einheit zur Erzeugung und Applikation eines Stimulationsdruckes 1 angefahren und über eine Stimulationszeitdauer T konstant gehalten werden muss. Zur Erhöhung der Genauigkeit können während des SD-Anstieges auch weitere IOP-Werte bestimmt werden, die in die Berechnung des individuellen Zusammenhanges SD = f(IOP) einbezogen werden.

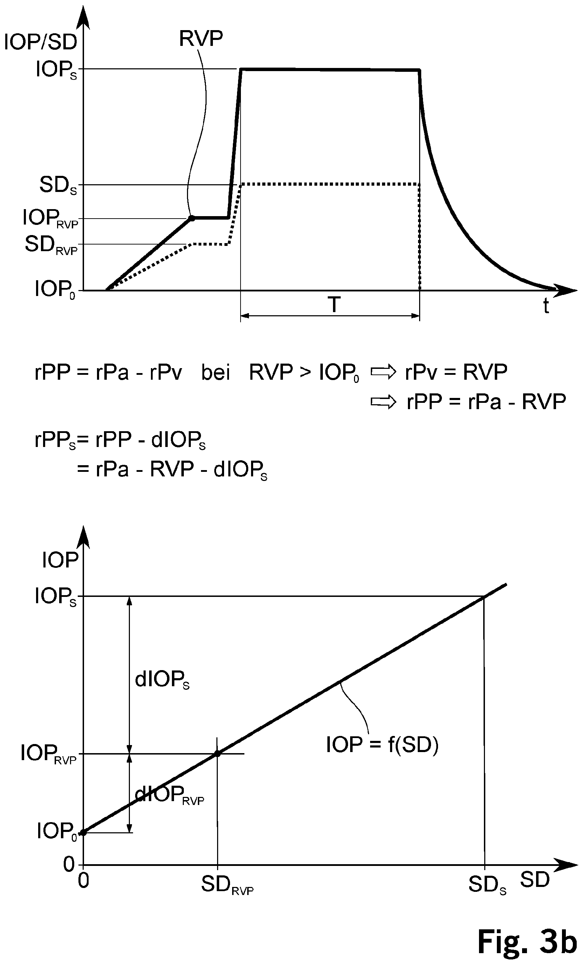

Im Fall des fehlenden spontanen Venenkollaps während der Baseline-Phase BP wird während der SD-Erhöhung durch die Signalanalyseeinheit 9 weiterhin der Eintritt des spontanen Venenkollaps überwacht, siehe hierzu **Fig. 3b.**

Sobald die Signalanalyseeinheit 9 den spontanen Venenkollaps auf dem Sehnervenkopf während der SD-Erhöhung erkennt, wird über die Rechen- und Steuereinheit 4 der dann dem retinalen Venendruck außerhalb des Augapfels RVP zugehörige erhöhte Intraokulardruckwert $IOP_{RVP}$ gemessen und aus der Differenz mit dem Ruhe-Intraokulardruck $IOP_0$ eine erfolgte spezifische Änderung des Intraokulardruckes $dIOP_{RVP}$ bestimmt, $dIOP_{RVP} = IOP_{RVP} - IOP_0$ bzw. $IOP_0 + dIOP_{RVP} = RVP$. Nach der beschriebenen Formel 2 und der individuellen Abhängigkeit SD = f(IOP) wird dann ein neu einzustellender Stimulationsdruckwert $SD_{Sneu} = f(dIOP_{RVP})$ berechnet, um den Intraokulardruck IOP, gleich dem RVP, durch Änderung um den vorgegebenen Änderungs-Intraokulardruckwert $dIOP_s$ auf einen neu errechneten Stimulations-Intraokulardruckwert $IOP_S$ zu erhöhen.

Trat der Venenkollaps bis zum Erreichen des vorgegebenen Stimulations-Intraokulardruckwertes $IOP_S$ bzw. bis zum Erreichen des zugehörigen Stimulationsdruckwertes $SD_s$ nicht auf und konnte folglich für den RVP kein Intraokulardruckwert $IOP_{RVP}$ bestimmt werden, wird der Stimulations-Intraokulardruckwert $IOP_S$ für den Fall IOP > RVP (Formel 1) eingestellt und es wird kein neuer Stimulations-Intraokulardruckwert $IOP_S$ berechnet und durch Einstellen eines neuen zugehörigen Stimulationsdruckwertes $SD_s$ erzeugt.

Nach dem Ablauf der Stimulationszeitdauer T wird die Stimulationsphase SP beendet und der Stimulationsdruck SD wird schlagartig auf Null zurückgefahren.

Die Aufzeichnung der Signale wird über eine Zeitspanne der Nachphase NP weiter fortgesetzt und dann beendet.

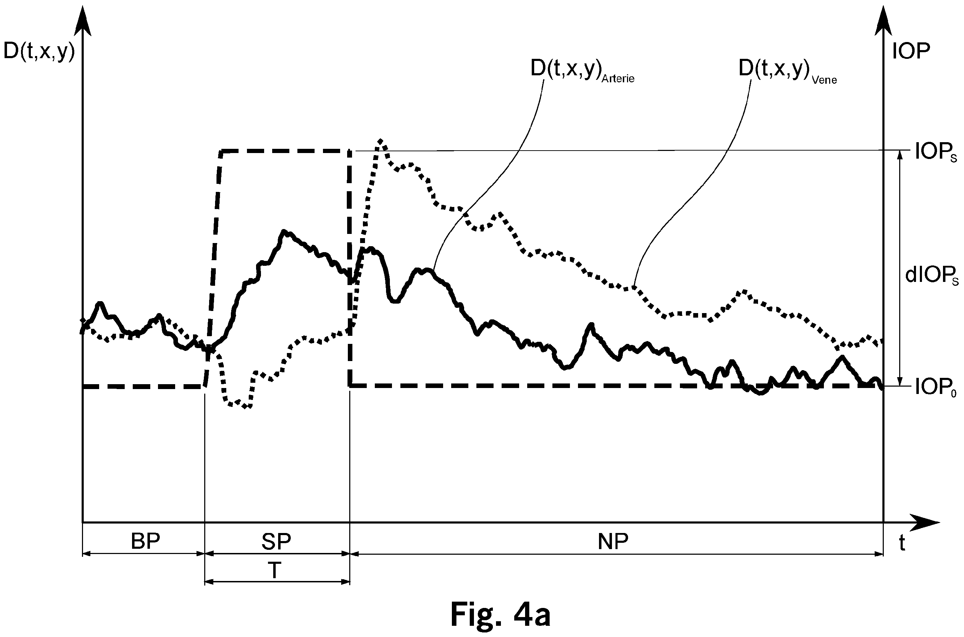

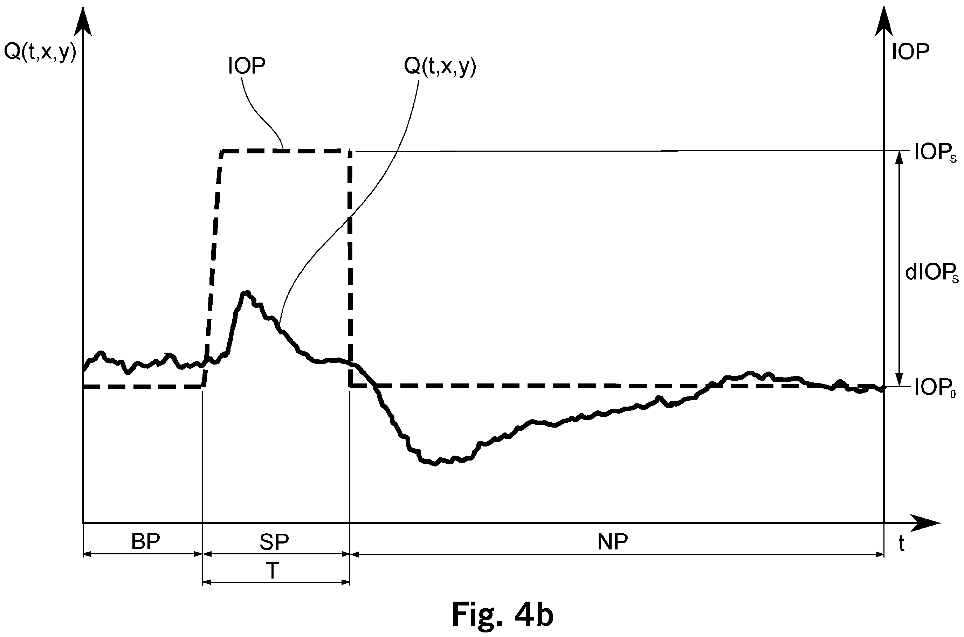

Die aufgezeichneten Signale werden als Beschreibung für die metabolische Autoregulation, wie z. B. in **Fig. 4a** Durchmessersignale D(t,x,y) oder in **Fig. 4b** Quotientensignale Q(t,x,y) gezeigt, ausgedruckt oder es werden charakteristische Messwerte, wie z. B. Minima und Maxima der Signale in der Stimulationsphase SP und aus der Nachphase NP, falschfarbenkodiert den Messorten zugeordnet und als Mappingbild einem der Videobilder der Netzhaut (Augenhintergrund) überlagert ausgegeben.

Die Reaktionen der großen Gefäße (Arterien, Venen) müssen nicht wie in dem voran beschriebenen Ausführungsbeispiel zwingend gemeinsam mit den Reaktionen der Kapillaren untersucht werden. Für bestimmte medizinische Fragestellungen ist auch die Untersuchung nur einer der Gefäßarten, Arterien, Kapillaren oder Venen, ausreichend.

Im Weiteren kann das Verfahren auch semiquantitativ erfolgen, indem der Untersucher U manuell die IOP-Werte bestimmt, wobei dann die entsprechenden längeren Pausen im SD-Anstieg realisiert werden müssen.

In einer weiteren Ausführungsform der Erfindung könnte die Bestimmung der individuellen Abhängigkeit SD = f(IOP) von der Stimulation gesondert erfolgen, um mögliche tonografische Effekte und Einflüsse auf die Gefäßreaktionen zu vermeiden.

Bezugszeichenliste

1       Einheit zur Erzeugung und Applikation eines Stimulationsdruckes
1.1     Druckapplikator
1.2     Druckerzeugungseinheit
1.3     Halterung
2       Bildgebungseinheit
3       Tonometer
4       Rechen- und Steuereinheit
5       Ein- und Ausgabeeinheit
7       Ergebnisbildspeichereinheit
8       Daten- und Bildauswerteeinheit
9       Signalanalyseeinheit
10      Einheit zur Bildung von spektral normierten Quotientensignalen
11      Einheit zur Bildung von Gefäßdurchmessersignalen

A     Auge
U     Untersucher

rPP       retinaler Perfusionsdruck(wert)
$rPP_s$   Stimulations-Perfusionsdruck
rPa       retinaler arterieller Blutdruck
rPv       retinaler venöser Blutdruck (innerhalb des Augapfels)
RVP       retinaler Venendruck(wert) außerhalb des Augapfels
SD        Stimulationsdruck(wert)
IOP       Intraokulardruck(wert)
$IOP_0$   Ruhe-Intraokulardruck(wert)
$dIOP_s$  vorgegebener Änderungs-Intraokulardruck(wert)
$IOP_S$   Stimulations-Intraokulardruckwert
$SD_s$    dem Stimulations-Intraokulardruckwert $IOP_S$ zugehöriger Stimulationsdruckwert
BP        Baseline-Phase
SP        Stimulationsphase
NP        Nachphase
T         Stimulationszeitdauer

Q(t,x,y)  (spektral normiertes) Quotientensignal
D(t,x,y)  Gefäßdurchmessersignal

**Patentansprüche**

**1.** Vorrichtung zur Untersuchung der metabolischen Autoregulation der Netzhautgefäße eines Auges (A) eines Patienten, enthaltend eine auf das Auge (A) wirkende Einheit zur Erzeugung und Applikation eines Stimulationsdruckes (1) und eine Bildgebungseinheit (2), wobei die Bildgebungseinheit (2) eine modifizierte Netzhautkamera mit digitalem Bildsensor ist, die von der Netzhaut eine Videosequenz von Bildern, denen jeweils zwei Farbkanäle zugeordnet sind, erzeugen kann, wobei die Vorrichtung eine Einheit zur Bildung von spektral normierten Quotientensignalen (10) enthält, die aus Intensitätssignalen der Farbkanäle Quotientensignale (Q(t,x,y)) ableiten kann, aus denen auf die Gefäßreaktion und damit die metabolische Autoregulation der Kapillaren der Netzhautgefäße geschlossen werden kann,
wobei die Vorrichtung ein Tonometer enthält, um einen Intraokulardruck (IOP) im Auge (A), der sich in Abhängigkeit von einem von der Einheit zur Erzeugung und Applikation eines Stimulationsdruckes (1) applizierten Stimulationsdruck (SD) ändert, zu messen, und dass die Einheit zur Erzeugung und Applikation eines Stimulationsdruckes (1) einen Sensor zur Messung des Stimulationsdruckes (SD) enthält, um jeweils einem gemessenen Intraokulardruckwert (IOP) und jedem Bild der Videosequenz einen Stimulationsdruckwert (SD) zuordnen zu können.

**2.** Vorrichtung zur Untersuchung der metabolischen Autoregulation der Netzhautgefäße eines Auges (A) eines Patienten nach Anspruch 1, **dadurch gekennzeichnet,**
**dass** die Einheit zur Erzeugung und Applikation eines Stimulationsdruckes (1) einen Druckapplikator (1.1) enthält, der am Kopf des Patienten ortsfest zum Auge (A) außerhalb der Hornhaut und eines Lichtweges der Bildgebungseinheit (2) druckfrei flächig an das Auge (A) anlegbar ist.

**3.** Vorrichtung zur Untersuchung der metabolischen Autoregulation der Netzhautgefäße eines Auges (A) eines Patienten nach Anspruch 1, **dadurch gekennzeichnet, dass** die Bildgebungseinheit (2) eine spektral modifizierte Netzhautkamera ist, die in ihrem Beleuchtungsstrahlengang einen doppelbandigen Bandpassfilter mit einem Spektralbereich im roten und einem Spektralbereich im grünen Licht aufweist.

**Claims**

**1.** Device for investigating the metabolic autoregulation of the retinal vessels of a patient's eye (A), comprising a unit acting on the eye (A) for generating and applying a stimulation pressure (1) and an imaging unit (2), wherein

the imaging unit (2) is a modified retinal camera with a digital image sensor which can generate a video sequence of images of the retina, to each of which two colour channels are assigned, the device containing a unit for forming spectrally normalized quotient signals (10) which can derive quotient signals (Q(t,x,y)) from intensity signals of the colour channels, from which quotient signals the vascular reaction and thus the metabolic autoregulation of the capillaries of the retinal vessels can be inferred, wherein the device includes a tonometer for measuring an intraocular pressure (IOP) in the eye (A) which changes in dependence on a stimulation pressure (SD) applied by the unit for generating and applying a stimulation pressure (1), and in that the unit for generating and applying a stimulation pressure (1) contains a sensor for measuring the stimulation pressure (SD) in order to be able to assign a stimulation pressure value (SD) to each measured intraocular pressure value (IOP) and to each image of the video sequence.

2. Device for examining the metabolic autoregulation of the retinal vessels of an eye (A) of a patient according to claim 1, **characterized in that** the unit for generating and applying a stimulation pressure (1) contains a pressure applicator (1.1) which can be applied to the patient's head in a stationary manner to the eye (A) outside the cornea and a light path of the imaging unit (2) in a pressure-free manner over the entire surface of the eye (A).

3. Device for examining the metabolic autoregulation of the retinal vessels of an eye (A) of a patient according to claim 1, **characterized in that** the imaging unit (2) is a spectrally modified retinal camera which has in its illumination beam path a double-band bandpass filter with a spectral range in red light and a spectral range in green light.

**Revendications**

1. Dispositif pour analyser l'autorégulation métabolique des vaisseaux rétiniens d'un oeil (A) d'un patient, contenant une unité de génération et application d'une pression de stimulation (1) agissant sur l'oeil (A) et une unité d'imagerie (2), dans lequel

l'unité d'imagerie (2) est une caméra rétinienne modifiée avec un capteur d'image numérique, qui peut produire à partir de la rétine une séquence vidéo d'images auxquelles sont associés respectivement deux canaux de couleur, le dispositif contenant une unité pour la formation de signaux de quotient (10) normalisés spectralement, qui peut déduire des signaux de quotient (Q(t,x,y)) à partir de signaux d'intensité des canaux de couleur, à partir desquels on peut conclure à la réaction vasculaire et donc à l'autorégulation métabolique des capillaires des vaisseaux rétiniens,
le dispositif comprenant un tonomètre pour mesurer une pression intraoculaire (IOP) dans l'oeil (A) qui varie en fonction d'une pression de stimulation (SD) appliquée par l'unité de génération et d'application d'une pression de stimulation (1), et en ce que l'unité de génération et d'application d'une pression de stimulation (1) contient un capteur pour mesurer la pression de stimulation (SD), afin de pouvoir associer une valeur de pression de stimulation (SD) à chaque valeur de pression intraoculaire (IOP) mesurée et à chaque image de la séquence vidéo.

2. Dispositif pour analyser l'autorégulation métabolique des vaisseaux rétiniens d'un oeil (A) d'un patient selon la revendication 1, **caractérisé en ce que** l'unité de génération et d'application d'une pression de stimulation (1) contient un applicateur de pression (1.1) qui peut être appliqué à plat sur l'oeil (A), sans pression, sur la tête du patient, en position fixe par rapport à l'oeil (A), en dehors de la cornée et d'un trajet lumineux de l'unité d'imagerie (2).

3. Dispositif pour analyser l'autorégulation métabolique des vaisseaux rétiniens d'un oeil (A) d'un patient selon la revendication 1, **caractérisé en ce que** l'unité d'imagerie (2) est une caméra rétinienne, modifiée spectralement, qui présente dans son trajet de faisceau d'éclairage un filtre passe-bande à double bande avec une zone spectrale dans la lumière rouge et une zone spectrale dans la lumière verte.

Fig. 1

EP 3 773 150 B1

Fig. 2

$$rPP = rPa - rPv \quad bei \quad RVP < IOP \implies rPv = IOP$$

$$\implies rPP = rPa - IOP$$

$$rPP_s = rPP - dIOP_s$$

$$= rPa - IOP_0 - dIOP_s$$

**Fig. 3a**

$$rPP = rPa - rPv \quad bei \quad RVP > IOP_0 \implies rPv = RVP$$
$$\implies rPP = rPa - RVP$$

$$rPP_S = rPP - dIOP_S$$
$$= rPa - RVP - dIOP_S$$

## Fig. 3b

**Fig. 4a**

Fig. 4b

EP 3 773 150 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **NAGEL, E. ; VILSER, W.** Autoregulative behavior of retinal arteries and veins during changes of perfusion pressure: a clinical study. *Graefe's Arch Clin Exp Ophthalmol,* 2004, vol. 242, 13-17